# EUROPEAN PATENT APPLICATION

(11) **EP 4 707 266 A1**
(43) Date of publication of application: **11.03.2026**
(21) Application number: 25795556.7
(22) Date of filing: 18.07.2025
(51) Int. Cl.: C07C 17/42, C07B 63/04, C07C 21/18

(54) **COMPOSITION CONTAINING HEXAFLUOROPROPENE**

(30) Priority: 26.07.2024 JP 2024121204; 26.07.2024 JP 2024121206; 26.07.2024 JP 2024121208
(71) Applicant: Daikin Industries, Ltd., Osaka-shi, Osaka 530-0001 (JP)
(72) Inventor: ETO, Yusuke, Osaka-Shi, Osaka 5300001 (JP); SUGIYAMA, Akinari, Osaka-Shi, Osaka 5300001 (JP); KUROKI, Yoshichika, Osaka-Shi, Osaka 5300001 (JP); NAKAMURA, Shingo, Osaka-Shi, Osaka 5300001 (JP)
(74) Representative: Hoffmann Eitle
(86) International application number: PCT/JP2025/025777
(87) International publication number: WO 2026/023579

(57) **Abstract**

The present disclosure aims to provide a novel hexafluoropropene-containing composition. A hexafluoropropene-containing composition is provided.

## Description

### Technical Field

The present disclosure relates to a hexafluoropropene-containing composition.

### Background Art

PLT 1 discloses a method of etching a silicon-based material such as a silicon oxide film and/or a low-dielectric constant film containing silicon by using hexafluoropropene.

### Citation List

### Patent Literature

PTL 1: WO0221586-A1

### Summary of Invention

### Technical Problem

The present disclosure aims to provide a novel hexafluoropropene-containing composition.

### Solution to Problem

The present disclosure encompasses the following features.

### Item 1.

A hexafluoropropene-containing composition comprising:
(1) hexafluoropropene;
(2) a hexafluoropropene-derived dimer and/or trimer; and
(3) at least one component selected from the group consisting of oxygen, hydrogen fluoride, and hydrogen chloride, wherein, based on the total amount of the composition,

when the composition contains oxygen as (3), the amount of the oxygen (3) is 0.05 ppm by volume or more and 10 ppm by volume or less,
when the composition contains hydrogen fluoride as (3), the amount of the hydrogen fluoride (3) is 0.05 ppm by volume or more and 5 ppm by volume or less, or
when the composition contains hydrogen chloride as (3), the amount of the hydrogen chloride (3) is 0.05 ppm by volume or more and 5 ppm by volume or less.

### Item 2.

The composition according to Item 1,
wherein, based on the total amount of the composition,
the hexafluoropropene (1) is present at 95 vol% to 99.99999 vol%, and
the hexafluoropropene-derived dimer and/or trimer (2) is present at 1 ppm by volume to 1,000 ppm by volume.

### Item 3.

A method for storing a hexafluoropropene-containing composition,
the composition comprising:
(1) hexafluoropropene;
(2) a hexafluoropropene-derived dimer and/or trimer; and
(3) at least one component selected from the group consisting of oxygen, hydrogen fluoride, and hydrogen chloride, wherein, based on the total amount of the composition,

when the composition contains oxygen as (3), the amount of the oxygen (3) is 0.05 ppm by volume or more and 10 ppm by volume or less,
when the composition contains hydrogen fluoride as (3), the amount of the hydrogen fluoride (3) is 0.05 ppm by volume or more and 5 ppm by volume or less, or
when the composition contains hydrogen chloride as (3), the amount of the hydrogen chloride (3) is 0.05 ppm by volume or more and 5 ppm by volume or less.

### Item 4.

The method according to Item 3,
wherein, based on the total amount of the composition,
the hexafluoropropene (1) is present at 95 vol% to 99.99999 vol%, and
the hexafluoropropene-derived dimer and/or trimer (2) is present at 1 ppm by volume to 1,000 ppm by volume.

### Item 5.

A method for suppressing a decrease in the purity of hexafluoropropene in a hexafluoropropene-containing composition, the composition comprising:
(1) hexafluoropropene;
(2) a hexafluoropropene-derived dimer and/or trimer; and
(3) at least one component selected from the group consisting of oxygen, hydrogen fluoride, and hydrogen chloride, wherein, based on the total amount of the composition,

when the composition contains oxygen as (3), the amount of the oxygen (3) is 0.05 ppm by volume or more and 10 ppm by volume or less,
when the composition contains hydrogen fluoride as (3), the amount of the hydrogen fluoride (3) is 0.05 ppm by volume or more and 5 ppm by volume or less, or
when the composition contains hydrogen chloride as (3), the amount of the hydrogen chloride (3) is 0.05 ppm by volume or more and 5 ppm by volume or less.

### Item 6.

The method according to Item 5,
wherein, based on the total amount of the composition,
the hexafluoropropene (1) is present at 95 vol% to 99.99999 vol%, and
the hexafluoropropene-derived dimer and/or trimer (2) is present at 1 ppm by volume to 1,000 ppm by volume.

The present disclosure discloses a method for stably transporting a composition containing a hexafluoropropene-containing composition (HFP) and a hexafluoropropene (HFP)-derived dimer and/or trimer. According to the present disclosure, the oxygen concentration, hydrogen fluoride concentration, or hydrogen chloride concentration in the HFP-containing composition is adjusted, which makes it possible to suppress the decomposition of HFP or HFP-derived dimer and/or trimer and suppress a decrease in the purity of HFP or its dimer and/or trimer during transportation of the HFP-containing composition in cylinders or the like, whereby the storage stability of the HFP-containing composition can be maintained.

The HFP-containing composition of the present disclosure contains an HFP-derived dimer and/or trimer. Thus, the composition has improved storage stability and can be stably transported by suppressing the decomposition of HFP or HFP-derived dimer and/or trimer even under harsh marine transportation conditions in containers or the like (e.g., conditions such as temperature increase during transportation).

### Advantageous Effects of Invention

The present disclosure can provide a novel hexafluoropropene-containing composition.

### Description of Embodiments

In the present specification, the terms "include," "contain," and variations thereof express concepts that encompass all of "comprise," "consist essentially of," and "consist of." In the present specification, when a numerical range is expressed as "A to B," the expression means A or more and B or less. In the present specification, when expressions such as "part(s)" and "%" are used, these mean parts by mass, parts by weight, mass%, or wt%.

### [1] Hexafluoropropene-containing composition

The hexafluoropropene (HFP)-containing composition of the present disclosure comprises:
(1) hexafluoropropene;
(2) a hexafluoropropene-derived dimer and/or trimer; and
(3) at least one component selected from the group consisting of oxygen, hydrogen fluoride, and hydrogen chloride, wherein, based on the total amount of the composition,

when the composition contains oxygen as (3), the amount of the oxygen (3) is 0.05 ppm by volume or more and 10 ppm by volume or less,
when the composition contains hydrogen fluoride as (3), the amount of the hydrogen fluoride (3) is 0.05 ppm by volume or more and 5 ppm by volume or less, or
when the composition contains hydrogen chloride as (3), the amount of the hydrogen chloride (3) is 0.05 ppm by volume or more and 5 ppm by volume or less.

Preferably, in the HFP-containing composition of the present disclosure,
based on the total amount of the composition,
the HFP (1) is present at 95 vol% to 99.99999 vol%, and
the HFP-derived dimer and/or trimer (2) is present at 1 ppm by volume to 1,000 ppm by volume.

The present disclosure discloses a method for stably transporting a composition containing HFP and an HFP-derived dimer and/or trimer. According to the present disclosure, the oxygen concentration, hydrogen fluoride concentration, or hydrogen chloride concentration in an HFP-containing composition is adjusted, which makes it possible to suppress the decomposition of HFP or HFP-derived dimer and/or trimer and suppress a decrease in the purity of HFP or HFP-derived dimer and/or trimer during transportation of the HFP-containing composition in cylinders or the like, whereby the storage stability of the HFP-containing composition can be maintained.

The HFP-containing composition of the present disclosure contains an HFP-derived dimer and/or trimer. Thus, the composition has improved storage stability and can be stably transported by suppressing the decomposition of HFP or HFP-derived dimer and/or trimer even under harsh marine transportation conditions in containers or the like (e.g., conditions such as temperature increase during transportation).

### (1) Hexafluoropropene

The HFP-containing composition of the present disclosure contains (1) hexafluoropropene (CF₃-CF=CF₂, also known as hexafluoropropylene, HFP) as a first component.

The HFP-containing composition of the present disclosure contains the first component "(1) HFP" as a main component. For improved storage stability and stable transportation by suppressing the decomposition of HFP or HFP-derived dimer and/or trimer even under harsh marine transportation conditions in containers or the like (e.g., conditions such as temperature increase during transportation), the amount of HFP in the HFP-containing composition based on the total amount (gas volume) of the HFP-containing composition is preferably 95 vol% to 99.99999 vol%, more preferably 95 vol% to 99.999 vol% or 96 vol% to 99.99 vol%, even more preferably 97 vol% to 99.95 vol%, and particularly preferably 98 vol% to 99.9 vol%.

### (2) Hexafluoropropene-derived dimer and/or trimer

The HFP-containing composition of the present disclosure contains a hexafluoropropene (HFP)-derived dimer and/or trimer (2) as a second component.

The HFP-containing composition of the present disclosure contains a hexafluoropropene-derived dimer and/or trimer as a second component. Thus, the composition has improved storage stability and can be stably transported by suppressing the decomposition of HFP or HFP-derived dimer and/or trimer even under harsh marine transportation conditions in containers or the like (e.g., conditions such as temperature increase during transportation).

In the HFP-containing composition of the present disclosure, the second component "hexafluoropropene (HFP)-derived dimer and/or trimer" is a product prepared from hexafluoropropene (C₃F₆), and is a component different from the first component "(1) hexafluoropropene (HFP)."

### Method for preparing dimer and/or trimer

### (a) Method for preparing HFP-derived dimer

The HFP-derived dimer includes the cis-isomer and the trans-isomer shown below. In the HFP-containing composition, either isomer may be used irrespective of its abundance ratio.

The HFP-derived dimer includes the cis-isomer and the trans-isomer, unless otherwise specified.

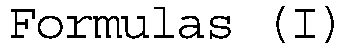

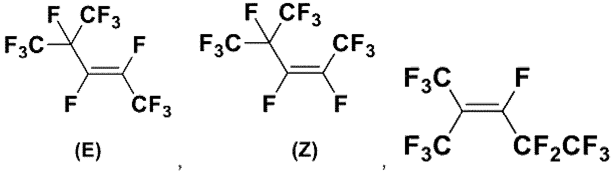

The hexafluoropropene dimer may include (E)-1,1,1,2,3,4,5,5,5-nonafluoro-4-(trifluoromethyl)-2-pentene, (Z)-1,1,1,2,3,4,5,5,5-nonafluoro-4-(trifluoromethyl)-2-pentene, and/or 1,1,1,3,4,4,5,5,5-nonafluoro-2-(trifluoromethyl)-2-pentene.

The HFP-derived dimer is prepared by the method described in JPH06-234672A, for example.

In the total amount of hexafluoropropene (HFP)-derived dimers (the sum amount of an HFP dimer mixture, below described as "the total amount of HFP dimers"), the mixing ratio of the above three compounds (total) based on the total amount of HFP dimers is preferably in the following order: 1 mass% or more, 10 mass% or more, 30 mass% or more, 40 mass% or more, and 45 mass% or more. A particularly preferred mixing ratio is 50 mass% or more. The mixing ratio of the above three compounds (total) based on the total amount of HFP dimers maybe 85 mass% or more. When the mixing ratio of the above three compounds (total) is high in the total amount of HFP dimers, the HFP dimer mixture has a lower viscosity.

The mixing ratio of the above three compounds (total) based on the total amount of HFP dimers is preferably in the following order: 99 mass% or less, 90 mass% or less, 85 mass% or less (or less than 85 mass%), 80 mass% or less, and 70 mass% or less. A particularly preferred mixing ratio is 60 mass% or less.

The mixing ratio of the above three compounds (total) based on the total amount of HFP dimers is adjusted to, for example, 10 mass% or more and 90 mass% or less, 10 mass% or more and 85 mass% or less, 10 mass% or more and less than 85 mass%, 20 mass% or more and less than 85 mass%, 30 mass% or more and 90 mass% or less, 30 mass% or more and less than 85 mass%, 35 mass% or more and 85 mass% or less, 35 mass% or more and 60 mass% or less, 40 mass% or more and less than 85 mass%, 40 mass% or more and 80 mass% or less, 50 mass% or more and less than 85 mass%, 50 mass% or more and 60 mass% or less, 55 mass% or more and 80 mass% or less, 60 mass% or more and 75 mass% or less, or 65 mass% or more and 70 mass% or less.

The mixing ratio of the above three compounds (total) based on the total amount of HFP dimers is preferably in the following order: 30 mass% or more and 90 mass% or less, 40 mass% or more and less than 85 mass, 40 mass% or more and 80 mass% or less, 45 mass% or more and 70 mass% or less. A particularly preferred mixing ratio 50 mass% or more and 60 mass% or less.

### (b) Method for preparing an HFP-derived trimer

The HFP-derived trimer includes the cis-isomer and the trans-isomer shown below. In the HFP-containing composition, either isomer may be used irrespective of its abundance ratio.

The compounds represented by Formulas (I) include E-diastereomer (I-(E)) and Z-diastereomer (I-(Z)), unless otherwise specified.

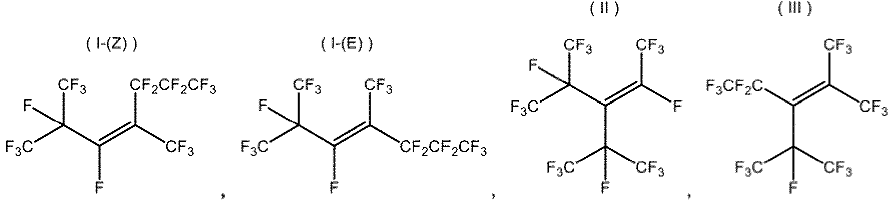

The HFP-derived trimer is appropriately selected depending on the desired abundance ratio and is produced by, for example, the method described in Chem Bar (1973), Vol. 106, pp. 2950-2959.

In the total amount of HFP-derived trimer (the sum amount of compounds represented by Formulas (I), (II), and (III) (an HFP trimer mixture), below described as "the total amount of HFP trimers"), the mixing ratio of the compounds represented by Formulas (I) based on the total amount of HFP trimers is preferably in the following order: 1 mass% or more, 10 mass% or more, 30 mass% or more, 40 mass% or more, and 45 mass% or more. A particularly preferred mixing ratio is 50 mass% or more. The mixing ratio of the compounds represented by Formulas (I) based on the total amount of HFP trimer may be 85 mass% or more. When the mixing ratio of the compounds represented by Formulas (I) is high in the total amount of HFP trimers, the HFP trimer mixture has a lower viscosity.

The mixing ratio of the compounds represented by Formulas (I) based on the total amount of HFP trimer is preferably in the following order: 99 mass% or less, 90 mass% or less, 85 mass% or less (or less than 85 mass%), 80 mass% or less, 70 mass% or less, and particularly preferably 60 mass% or less.

The mixing ratio of the compounds represented by Formulas (I) is adjusted as follows, for example, based on the total amount of HFP trimer: 10 mass% or more and 90 mass% or less, 10 mass% or more and 85 mass% or less, 10 mass% or more and less than 85 mass%, 20 mass% or more and less than 85 mass%, 30 mass% or more and 90 mass% or less, 30 mass% or more and less than 85 mass%, 35 mass% or more and 85 mass% or less, 35 mass% or more and 60 mass% or less, 40 mass% or more and less than 85 mass%, 40 mass% or more and 80 mass% or less, 50 mass% or more and less than 85 mass%, 50 mass% or more and 60 mass% or less, 55 mass% or more and 80 mass% or less, 60 mass% or more and 75 mass% or less, and 65 mass% or more and 70 mass% or less.

The mixing ratio of the compounds represented by Formulas (I) based on the total amount of HFP trimer is preferably in the following order: 30 mass% or more and 90 mass% or less, 40 mass% or more and less than 85 mass%, 40 mass% or more and 80 mass% or less, 45 mass% or more and 70 mass% or less, and particularly preferably 50 mass% or more and 60 mass% or less.

### (c) Amount of dimer and/or trimer

In the HFP-containing composition, one HFP-derived dimer and/or trimer may be used alone or two or more thereof may be used (blended) as a mixture.

For improved storage stability and stable transportation by suppressing the decomposition of HFP or HFP-derived dimer and/or trimer even under harsh marine transportation conditions in containers or the like (e.g., conditions such as temperature increase during transportation), the amount of HFP-derived dimer and/or trimer in the HFP-containing composition based on the total amount (gas volume) is preferably 1 ppm by volume (ppm volume) to 1,000 ppm by volume (ppm volume), more preferably 10 ppm by volume to 500 ppm by volume, even more preferably 10 ppm by volume to 300 ppm by volume, and particularly preferably 50 ppm by volume to 300 ppm by volume.

### (3) Oxygen, hydrogen fluoride, and hydrogen chloride

The HFP-containing composition of the present disclosure contains, as a third component, (3) at least one component selected from the group consisting of oxygen (O₂), hydrogen fluoride (HF), and hydrogen chloride (HCl).

### (a) Oxygen (O₂)

The HFP-containing composition of the present disclosure contains (3) oxygen (O₂) as a third component.

The amount of oxygen (O₂) in the HFP-containing composition based on the total amount (gas volume) of the HFP-containing composition is 0.05 ppm by volume or more and 10 ppm by volume or less. The O₂ concentration in the HFP-containing composition is adjusted within the above range, which makes it possible to suppress the decomposition of HFP or HFP-derived dimer and/or trimer and suppress a decrease in the purity of HFP or HFP-derived dimer and/or trimer in the HFP-containing composition during transportation of the HFP-containing composition in cylinders or the like, whereby the storage stability of the HFP-containing composition can be well maintained. The HFP-containing composition can be stably transported by suppressing the decomposition of HFP or HFP-derived dimer and/or trimer even under harsh marine transportation conditions in containers or the like (e.g., conditions such as temperature increase during transportation).

The amount of oxygen (O₂) in the HFP-containing composition based on the total amount (gas volume) of the HFP-containing composition is preferably 0.1 ppm by volume (ppm volume) to 10 ppm by volume (ppm volume), and more preferably 0.1 ppm by volume to 5 ppm by volume.

### (b) Hydrogen fluoride (HF)

The HFP-containing composition of the present disclosure contains (3) hydrogen fluoride (HF) as a third component.

The amount of hydrogen fluoride (HF) in the HFP-containing composition based on the total amount (gas volume) of the HFP-containing composition is 0.05 ppm by volume or more and 5 ppm by volume or less. The HF concentration in the HFP-containing composition is adjusted within the above range, which makes it possible to suppress the decomposition of HFP or HFP-derived dimer and/or trimer and suppress a decrease in the purity of HFP or HFP-derived dimer and/or trimer in the HFP-containing composition during transportation of the HFP-containing composition in cylinders or the like, whereby the storage stability of the HFP-containing composition can be well maintained. The HFP-containing composition can be stably transported by suppressing the decomposition of HFP or HFP-derived dimer and/or trimer even under harsh marine transportation conditions in containers or the like (e.g., conditions such as temperature increase during transportation).

The amount of hydrogen fluoride (HF) in the HFP-containing composition based on the total amount (gas volume) of the HFP-containing composition is preferably 0.1 ppm by volume (ppm volume) to 5 ppm by volume (ppm volume), more preferably 0.1 ppm by volume to 3 ppm by volume, and even more preferably 0.1 ppm by volume to 1 ppm by volume.

### (c) Hydrogen chloride (HCl)

The HFP-containing composition of the present disclosure contains (3) hydrogen chloride (HCl) as a third component.

The amount of hydrogen chloride (HCl) in the HFP-containing composition based on the total amount (gas volume) of the HFP-containing composition is 0.05 ppm by volume or more and 5 ppm by volume or less. The HCl concentration in the HFP-containing composition is adjusted within the above range, which makes it possible to suppress the decomposition of HFP or its dimer and/or trimer and suppress a decrease in the purity of HFP or HFP-derived dimer and/or trimer in the HFP-containing composition during transportation of the HFP-containing composition in cylinders or the like, whereby the storage stability of the HFP-containing composition can be well maintained. The HFP-containing composition can be stably transported by suppressing the decomposition of HFP or HFP-derived dimer and/or trimer even under harsh marine transportation conditions in containers or the like (e.g., conditions such as temperature increase during transportation).

The amount of hydrogen chloride (HCl) in the HFP-containing composition based on the total amount (gas volume) of the HFP-containing composition is preferably 0.1 ppm by volume (ppm volume) to 5 ppm by volume (ppm volume), more preferably 0.1 ppm by volume to 3 ppm by volume, and even more preferably 0.1 ppm by volume to 1 ppm by volume.

### [2] Method for storing hexafluoropropene-containing composition

The present disclosure encompasses a method for storing a hexafluoropropene (HFP)-containing composition and a method for suppressing a decrease in the purity of HFP in an HFP-containing composition.

In the method for storing an HFP-containing composition or the method for suppressing a decrease in the purity of HFP, the HFP-containing composition comprises:
(1) hexafluoropropene (HFP);
(2) a hexafluoropropene (HFP)-derived dimer and/or trimer; and
(3) at least one component selected from the group consisting of oxygen, hydrogen fluoride, and hydrogen chloride, wherein, based on the total amount of the composition,

when the composition contains oxygen as (3), the amount of the oxygen (3) is 0.05 ppm by volume or more and 10 ppm by volume or less,
when the composition contains hydrogen fluoride as (3), the amount of the hydrogen fluoride (3) is 0.05 ppm by volume or more and 5 ppm by volume or less, or
when the composition contains hydrogen chloride as (3), the amount of the hydrogen chloride (3) is 0.05 ppm by volume or more and 5 ppm by volume or less.

Preferably, in the HFP-containing composition, based on the total amount of the composition,
the HFP (1) is present at 95 vol% to 99.99999 vol%, and
the HFP-derived dimer and/or trimer (2) is present at 1 ppm by volume to 1,000 ppm by volume.

The details of the HFP-containing composition are as described in section [1] Hexafluoropropene-containing composition.

The present disclosure discloses a method for stably transporting a composition containing HFP and HFP-derived dimer and/or trimer. According to the present disclosure, the oxygen (O₂) concentration, hydrogen fluoride (HF) concentration, or hydrogen chloride (HCl) concentration in the HFP-containing composition is adjusted, which makes it possible to suppress the decomposition of HFP or HFP-derived its dimer and/or trimer and suppress a decrease in the purity of HFP or HFP-derived dimer and/or trimer in the HFP-containing composition during transportation of the HFP-containing composition in cylinders or the like, whereby the storage stability of the HFP-containing composition can be well maintained.

The HFP-containing composition of the present disclosure contains HFP-derived dimer and/or trimer and suppresses the generation of an HFP-derived decomposed product or HFP-derived dimer- and/or trimer-derived decomposed product. Thus, the composition has improved storage stability and can be stably transported by suppressing the decomposition of HFP or its dimer and/or trimer even under harsh marine transportation conditions in containers or the like (e.g., conditions such as temperature increase during transportation).

### [3] Application of hexafluoropropene-containing composition

The present disclosure encompasses a method for etching a semiconductor manufacturing substrate using, as an etching gas, the hexafluoropropene (HFP)-containing composition of the present disclosure, which is supplied as a plasma-generating gas.

A silicon oxide film (SiO₂ film) or a silicon-based material having a silicon nitride film (SiN film) formed thereon can be etched using a gas plasma of the HFP-containing composition (etching gas). The silicon-based material to be etched is preferably a semiconductor manufacturing substrate.

When hole etching is performed in a silicon oxide film (SiO₂ film) or a silicon-based material having a silicon nitride film (SiN film) formed thereon using a gas plasma generated from the HFP-containing composition, good etching can be achieved in which the processed hole shape is well maintained. The conditions for the etching method (in particular, the dry etching method) are the same as those for the conventional method.

### Examples

The present invention is specifically described below with reference to Examples and Comparative Examples, which do not limit the present invention.

### [1-1] Storage stability of hexafluoropropene-containing composition

### Example 1-1

Hexafluoropropene: HFP
Hexafluoropropene-derived dimer and/or trimer: dimer trimer Oxygen: O₂

According to a conventional method, HFP was purified, the dimer and/or trimer concentration was adjusted to predetermined levels (50 ppm by volume, 20 ppm by volume, and 5 ppm by volume), and the O₂ concentration was adjusted to predetermined levels (100 ppm by volume, 10 ppm by volume, 5 ppm by volume, and 1 ppm by volume), whereby HFP-containing compositions were prepared.

The dimer and/or trimer concentration (ppm by volume) in each HFP-containing composition was analyzed by gas chromatography (GC analysis).

The O₂ concentration (ppm by volume) in each HFP-containing composition was analyzed by GC.

The HFP-containing compositions were stored at a constant temperature (20°C or 50°C). After fixed periods (30 days, 90 days, and 180 days), the gas phase was extracted, and a hexafluoropropene (HFP)-derived decomposed product was analyzed by GC analysis (ppm by volume).

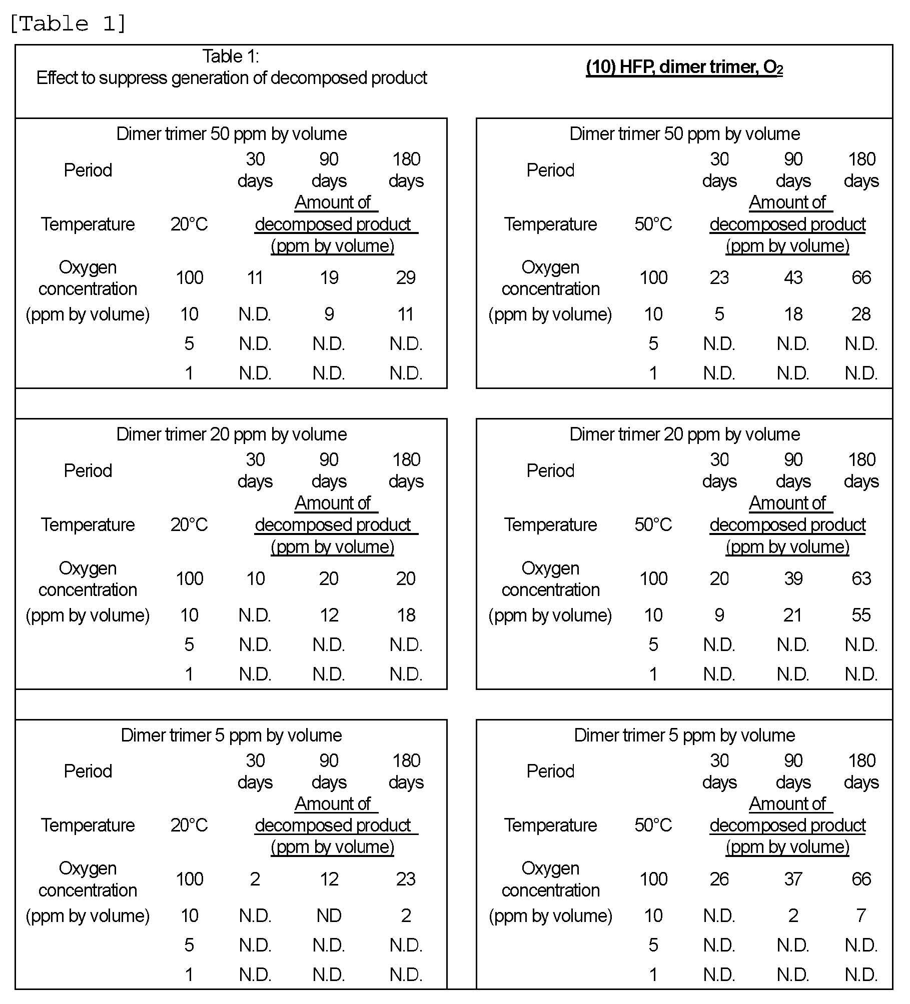

The compositions containing HFP and HFP-derived dimer and/or trimer (50 ppm by volume, 20 ppm by volume, and 5 ppm by volume) of the Example in which the O₂ concentration was adjusted (10 ppm by volume or less) successfully suppressed the generation of an HFP-derived decomposed product or a dimer- and/or trimer-derived decomposed product even after the fixed periods (30 days, 90 days, and 180 days) of storage at a constant temperature (20°C or 50°C).

### [1-2] Study of lower limit amount of oxygen (O₂) in composition

### Example 1-2

According to a conventional method, a composition containing HFP and HFP-derived dimer and/or trimer (5 ppm by volume) was prepared, and the O₂ concentration was adjusted to predetermined levels (0.1 ppm by volume, 0.01 ppm by volume), whereby compositions containing O₂ and a dimer and/or trimer were prepared.

The O₂ concentration (ppm by volume) in each composition containing HFP and dimer and/or trimer (5 ppm by volume) was analyzed by GC.

The compositions containing HFP and HFP-derived dimer and/or trimer (5 ppm by volume) were stored at a constant temperature (20°C, 50°C). After fixed periods (30 days, 90 days, and 180 days), the gas phase was extracted and analyzed by GC (ppm by volume).

**[Table 2]**

| Table 2: Study of lower limit amount of oxygen | | | | |
|---|---|---|---|---|
| O₂ concentration (ppm by volume) | | Decomposed product (ppm by volume) | | |
| 20°C | | 30 days | 90 days | 180 days |
| Example 1-2-1 | 0.1 | N.D. | N.D. | N.D. |
| Comparative Example 1-1-1 | 0.01 | 0.03 | 0.04 | 0.05 |
| | | | | |

| O₂ concentration (ppm by volume) | | Decomposed product (ppm by volume) | | |
|---|---|---|---|---|
| 50°C | | 30 days | 90 days | 180 days |
| Example 1-2-2 | 0.1 | N.D. | N.D. | N.D. |
| Comparative Example 1-1-2 | 0.01 | 0.04 | 0.06 | 0.07 |

In the composition in which the O₂ concentration was 0.1 ppm by volume, no decomposed product was observed. In the composition in which the O₂ concentration was 0.01 ppm by volume, a decomposed product was detected.

### [2-1] Storage stability of hexafluoropropene-containing composition

### Example 2-1

Hexafluoropropene: HFP
Hexafluoropropene-derived dimer and/or trimer: dimer trimer Hydrogen fluoride: HF

According to a conventional method, HFP was purified, the dimer and/or trimer concentration was adjusted to predetermined levels (50 ppm by volume, 20 ppm by volume, and 5 ppm by volume), and the HF concentration was adjusted to predetermined levels (10 ppm by volume, 5 ppm by volume, 1 ppm by volume, and 0.1 ppm by volume), whereby HFP-containing compositions were prepared.

The dimer and/or trimer concentration (ppm by volume) in each HFP-containing composition was analyzed by gas chromatography (GC analysis).

The HF concentration (ppm by volume) in each HFP-containing composition was analyzed by a Fourier transform infrared spectrophotometer (FT-IR) to measure the initial concentration.

The HFP-containing compositions were stored at a constant temperature (20°C or 50°C). After fixed periods (30 days, 90 days, and 180 days), the gas phase was extracted, and a hexafluoropropene (HFP)-derived decomposed product was analyzed by GC analysis (ppm by volume).

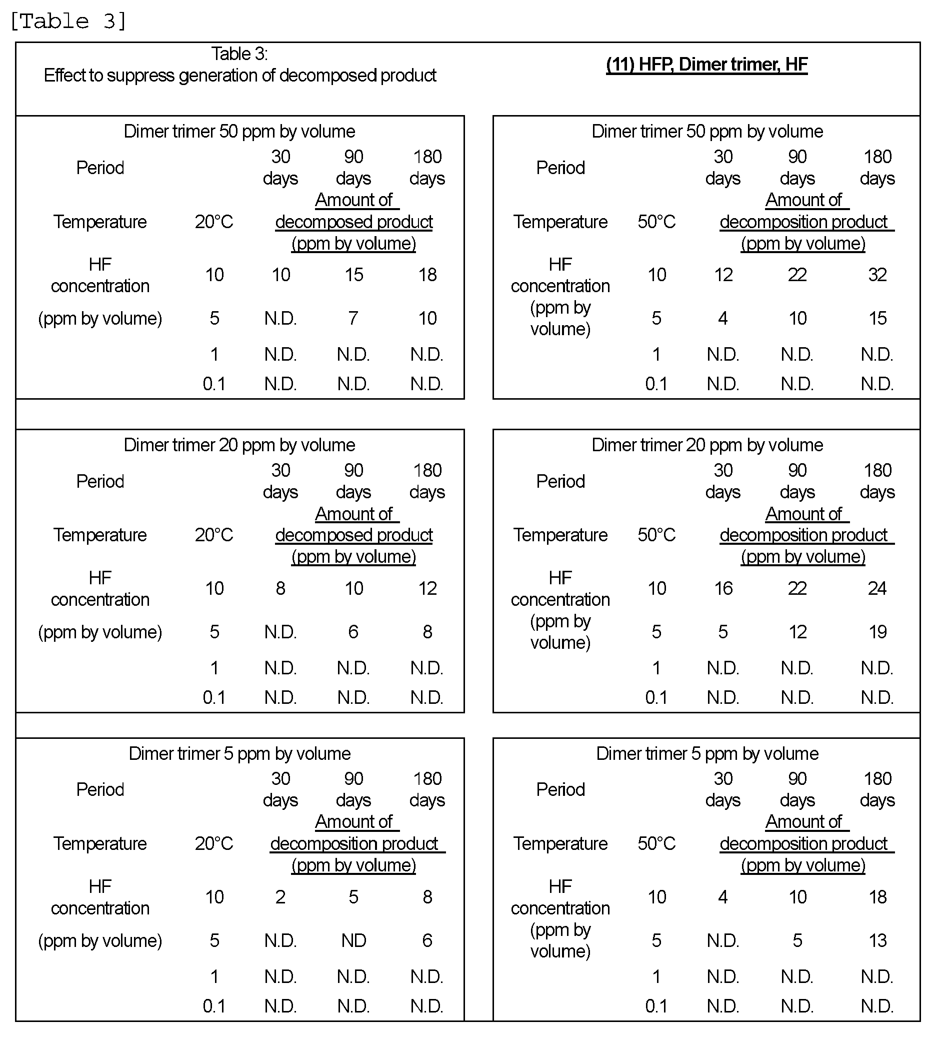

The compositions containing HFP and HFP-derived dimer and/or trimer (50 ppm by volume, 20 ppm by volume, and 5 ppm by volume) of the Example in which the HF concentration was adjusted (5 ppm by volume or less) successfully suppressed the generation of an HFP-derived decomposed product or a dimer- and/or trimer-derived decomposed product even after the fixed periods (30 days, 90 days, and 180 days) of storage at a constant temperature (20°C or 50°C).

### [2-2] Study of lower limit amount of hydrogen fluoride (HF) in composition

### Example 2-2

According to a conventional method, a composition containing HFP and dimer and/or trimer (5 ppm by volume) was prepared, and the HF concentration was adjusted to predetermined levels (0.1 ppm by volume, 0.01 ppm by volume), whereby compositions containing HF and a dimer and/or trimer were prepared.

The HF concentration (ppm by volume) in each composition containing HFP and dimer and/or trimer (5 ppm by volume) was analyzed by GC.

The compositions containing HFP and dimer and/or trimer (5 ppm by volume) were stored at a constant temperature (20°C, 50°C). After fixed periods (30 days, 90 days, and 180 days), the gas phase was extracted and analyzed by GC (ppm by volume).

**[Table 4]**

| Table 4: Study of lower limit amount of hydrogen fluoride | | | | |
|---|---|---|---|---|
| HF concentration (ppm by volume) | | Decomposed product (ppm by volume) | | |
| 20°C | | 30 days | 90 days | 180 days |
| Example 2-2-1 | 0.1 | N.D. | N.D. | N.D. |
| Comparative Example 2-1-1 | 0.01 | 0.1 | 0.3 | 0.5 |
| | | | | |

| HF concentration (ppm by volume) | | Decomposed product (ppm by volume) | | |
|---|---|---|---|---|
| 50°C | | 30 days | 90 days | 180 days |
| Example 2-2-2 | 0.1 | N.D. | N.D. | N.D. |
| Comparative Example 2-1-2 | 0.01 | 0.2 | 0.5 | 0.6 |

In the composition in which the HF concentration was 0.1 ppm by volume, no decomposed product was observed. In the composition in which the HF concentration was 0.01 ppm by volume, a decomposed product was detected.

### [3-1] Storage stability of hexafluoropropene-containing composition

### Example 3-1

Hexafluoropropene: HFP
Hexafluoropropene-derived dimer and/or trimer: dimer trimer Hydrogen chloride: HCl

According to a conventional method, HFP was purified, the dimer and/or trimer concentration was adjusted to predetermined levels (50 ppm by volume, 20 ppm by volume, and 5 ppm by volume), and the HCl concentration was adjusted to predetermined levels (10 ppm by volume, 5 ppm by volume, 1 ppm by volume, and 0.1 ppm by volume), whereby HFP-containing compositions were prepared.

The dimer and/or trimer concentration (ppm by volume) in each HFP-containing composition was analyzed by gas chromatography (GC analysis).

The HCL concentration (ppm by volume) in each HFP-containing composition was analyzed by a Fourier transform infrared spectrophotometer (FT-IR) to measure the initial concentration.

The HFP-containing compositions were stored at a constant temperature (20°C or 50°C). After fixed periods (30 days, 90 days, and 180 days), the gas phase was extracted, and a hexafluoropropene (HFP)-derived decomposed product was analyzed by GC analysis (ppm by volume).

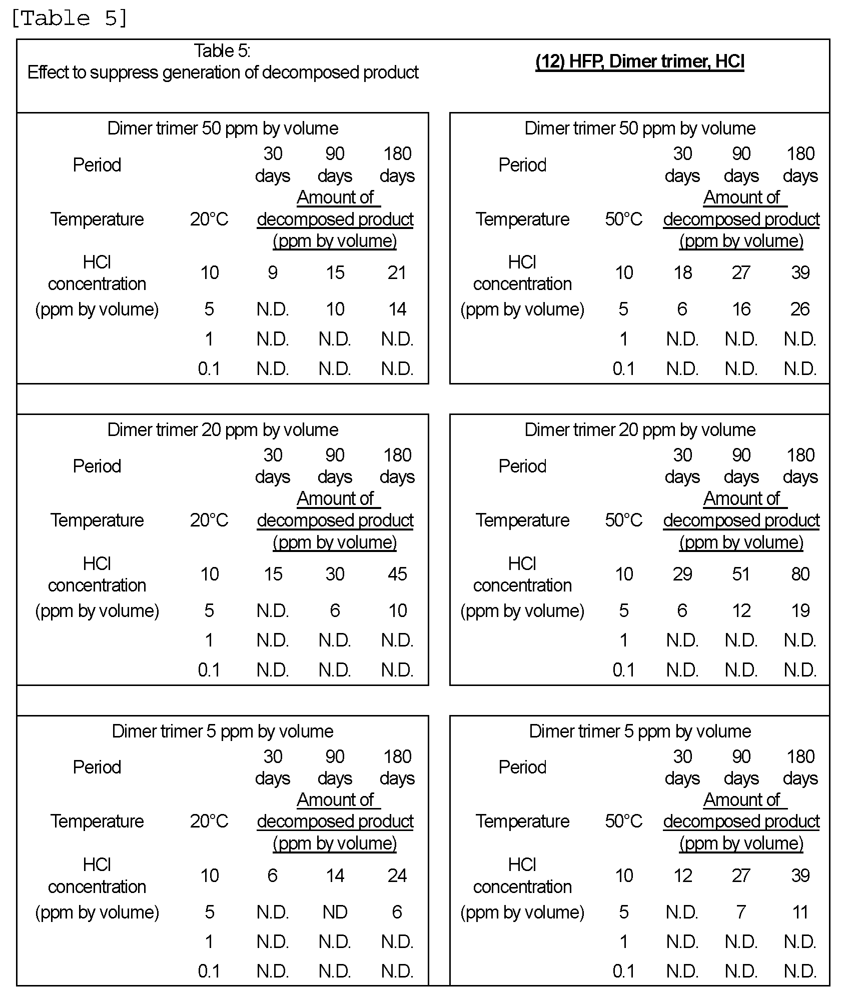

The compositions containing HFP and its dimer and/or trimer (50 ppm by volume, 20 ppm by volume, and 5 ppm by volume) of the Example in which the HCl concentration was adjusted (5 ppm by volume or less) successfully suppressed the generation of an HFP-derived decomposed product or a dimer- and/or trimer-derived decomposed product even after the fixed periods (30 days, 90 days, and 180 days) of storage at a constant temperature (20°C or 50°C) .

### [3-2] Study of lower limit amount of hydrogen chloride (HCl) in composition

### Example 3-2

According to a conventional method, a composition containing HFP and its dimer and/or trimer (5 ppm by volume) was prepared, and the HCl concentration was adjusted to predetermined levels (0.1 ppm by volume, 0.01 ppm by volume), whereby compositions containing HCl and a dimer and/or trimer were prepared.

The HCL concentration (ppm by volume) in each composition containing HFP and dimer and/or trimer (5 ppm by volume) was analyzed by GC.

The compositions containing HFP and dimer and/or trimer (5 ppm by volume) were stored at a constant temperature (20°C, 50°C). After fixed periods (30 days, 90 days, and 180 days), the gas phase was extracted and analyzed by GC (ppm by volume).

**[Table 6]**

| Table 6: Study of lower limit amount of hydrogen chloride | | | | |
|---|---|---|---|---|
| HCl concentration (ppm by volume) | | Decomposed product (ppm by volume) | | |
| 20°C | | 30 days | 90 days | 180 days |
| Example 3-2-1 | 0.1 | N.D. | N.D. | N.D. |
| Comparative Example 3-1-1 | 0.01 | 0.1 | 0.2 | 0.5 |
| | | | | |

| HCl concentration (ppm by volume) | | Decomposed product (ppm by volume) | | |
|---|---|---|---|---|
| 50°C | | 30 days | 90 days | 180 days |
| Example 3-2-1 | 0.1 | N.D. | N.D. | N.D. |
| Comparative Example 3-1-2 | 0.01 | 0.2 | 0.3 | 0.6 |

In the composition in which the HCl concentration was 0.1 ppm by volume, no decomposed product was observed. In the composition in which the HCl concentration was 0.01 ppm by volume, a decomposed product was detected.

### [4] Industrial Applicability

The present disclosure discloses a method for stably transporting a composition containing HFP and a hexafluoropropene-derived dimer and/or trimer. According to the present disclosure, the oxygen (O₂) concentration, hydrogen fluoride (HF) concentration, or hydrogen chloride (HCl) concentration in the HFP-containing composition is adjusted, which makes it possible to suppress the decomposition of HFP or HFP-derived dimer and/or trimer and suppress a decrease in the purity of HFP or HFP-derived dimer and/or trimer in the HFP-containing composition during transportation of the HFP-containing composition in cylinders or the like, whereby the storage stability of the HFP-containing composition can be well maintained.

The HFP-containing composition of the present disclosure contains a hexafluoropropene-derived dimer and/or trimer and suppresses the generation of an HFP-derived decomposed product or HFP-derived dimer- and/or trimer-derived decomposed product. Thus, the composition has improved storage stability and can be stably transported by suppressing the decomposition of HFP or HFP-derived dimer and/or trimer even under harsh marine transportation conditions in containers or the like (e.g., conditions such as temperature increase during transportation).

## Claims

1. A hexafluoropropene-containing composition comprising:
(1) hexafluoropropene;
(2) a hexafluoropropene-derived dimer and/or trimer; and
(3) at least one component selected from the group consisting of oxygen, hydrogen fluoride, and hydrogen chloride, wherein, based on the total amount of the composition,
when the composition contains oxygen as (3), the amount of the oxygen (3) is 0.05 ppm by volume or more and 10 ppm by volume or less,
when the composition contains hydrogen fluoride as (3), the amount of the hydrogen fluoride (3) is 0.05 ppm by volume or more and 5 ppm by volume or less, or
when the composition contains hydrogen chloride as (3), the amount of the hydrogen chloride (3) is 0.05 ppm by volume or more and 5 ppm by volume or less.

2. The composition according to claim 1,
wherein, based on the total amount of the composition,
the hexafluoropropene (1) is present at 95 vol% to 99.99999 vol%, and
the hexafluoropropene-derived dimer and/or trimer (2) is present at 1 ppm by volume to 1,000 ppm by volume.

3. A method for storing a hexafluoropropene-containing composition,
the composition comprising:
(1) hexafluoropropene;
(2) a hexafluoropropene-derived dimer and/or trimer; and
(3) at least one component selected from the group consisting of oxygen, hydrogen fluoride, and hydrogen chloride, wherein, based on the total amount of the composition,
when the composition contains oxygen as (3), the amount of the oxygen (3) is 0.05 ppm by volume or more and 10 ppm by volume or less,
when the composition contains hydrogen fluoride as (3), the amount of the hydrogen fluoride (3) is 0.05 ppm by volume or more and 5 ppm by volume or less, or
when the composition contains hydrogen chloride as (3), the amount of the hydrogen chloride (3) is 0.05 ppm by volume or more and 5 ppm by volume or less.

4. The method according to claim 3,
wherein, based on the total amount of the composition,
the hexafluoropropene (1) is present at 95 vol% to 99.99999 vol%, and
the hexafluoropropene-derived dimer and/or trimer (2) is present at 1 ppm by volume to 1,000 ppm by volume.

5. A method for suppressing a decrease in the purity of hexafluoropropene in a hexafluoropropene-containing composition, the composition comprising:
(1) hexafluoropropene;
(2) a hexafluoropropene-derived dimer and/or trimer; and
(3) at least one component selected from the group consisting of oxygen, hydrogen fluoride, and hydrogen chloride, wherein, based on the total amount of the composition,
when the composition contains oxygen as (3), the amount of the oxygen (3) is 0.05 ppm by volume or more and 10 ppm by volume or less,
when the composition contains hydrogen fluoride as (3), the amount of the hydrogen fluoride (3) is 0.05 ppm by volume or more and 5 ppm by volume or less, or
when the composition contains hydrogen chloride as (3), the amount of the hydrogen chloride (3) is 0.05 ppm by volume or more and 5 ppm by volume or less.

6. The method according to claim 5,
wherein, based on the total amount of the composition,
the hexafluoropropene (1) is present at 95 vol% to 99.99999 vol%, and
the hexafluoropropene-derived dimer and/or trimer (2) is present at 1 ppm by volume to 1,000 ppm by volume.
